# EUROPEAN PATENT SPECIFICATION

(11) **EP 4 210 588 B1**
(45) Date of publication and mention of the grant of the patent: **26.02.2025**
(21) Application number: 21777206.0
(22) Date of filing: 07.09.2021
(51) Int. Cl.: A61B 8/08

(54) **SYSTEMS AND METHODS FOR MEASURING CARDIAC STIFFNESS**
VORRICHTUNGEN UND VERFAHREN ZUR MESSUNG DER HERZSTEIFHEIT
DISPOSITFS ET PROCÉDÉS POUR LA MESURE DE RAIDEUR DU COEUR

(30) Priority: 07.09.2020 US 202063075293 P
(43) Date of publication of application: 19.07.2023
(73) Proprietor: Koninklijke Philips N.V., 5656 AG Eindhoven (NL)
(72) Inventor: SADEGHI, Seyedali, 5656 AE Eindhoven (NL); VIGNON, Francois, Guy, Gerard, Marie, 5656 AE Eindhoven (NL); RAFTER, Patrick, Gabriels, 5656 AE Eindhoven (NL)
(74) Representative: Philips Intellectual Property & Standards
(86) International application number: PCT/EP2021/074530
(87) International publication number: WO 2022/049299

(56) References cited:
- EP-A1- 2 082 688
- ENGEL AARON J ET AL: "Cardiac atrial kick shear wave elastography with ultrafast diverging wave imaging: An in vivo pilot study", 2017 IEEE INTERNATIONAL ULTRASONICS SYMPOSIUM (IUS), IEEE, 6 September 2017 (2017-09-06), pages 1 - 4, XP033245848, DOI: 10.1109/ULTSYM.2017.8092742
- KVÅLE KAJA F ET AL: "Detection of Tissue Fibrosis using Natural Mechanical Wave Velocity Estimation: Feasibility Study", ULTRASOUND IN MEDICINE AND BIOLOGY, NEW YORK, NY, US, vol. 46, no. 9, 4 June 2020 (2020-06-04), pages 2481 - 2492, XP086244019, ISSN: 0301-5629, [retrieved on 20200604], DOI: 10.1016/J.ULTRASMEDBIO.2020.04.022

## Description

### TECHNICAL FIELD

The present disclosure pertains to imaging systems and methods for cardiac measurements. **In** particular, the disclosure pertains to semi-automated imaging systems and methods for measuring cardiac stiffness.

### BACKGROUND

Heart Failure (the inability of the heart to provide sufficient cardiac output while maintaining normal filling pressures) is affecting at least 26 million people worldwide and it is estimated to increase by 46% by 2030. Two types of heart failure exist: heart failure with reduced ejection fraction (HFrEF) and heart failure with preserved ejection fraction (HFpEF). The latter, HFpEF, makes up 50% of heart failure cases, and is characterized by impaired relaxation of the left ventricle (LV) during diastole and increased filling pressures caused by altered LV mechanical properties, most notably higher stiffness. While there are current guidelines for the diagnosis of HFpEF (history and physical examination, echocardiography, and cardiac catheterization if necessary), these guidelines are complicated and rarely followed, and even with them identifying the root cause of HFpEF is challenging. Conditions such as hypertension, hypertrophic cardiomyopathy, and cardiac amyloid can produce HFpEF. Typical ultrasound imaging (e.g., B-mode) is the primary imaging modality for HFpEF. However, it cannot be directly used for differential diagnosis.

Elastography has shown to be promising tool to non-invasively measure the stiffness of soft tissues. Different elastography techniques have been presented in order to non-invasively assess myocardial stiffness using ultrasound. All approaches capture the tissue's transient response to a local displacement in order to estimate the stiffness of the myocardium. However, they fundamentally differ on the excitation source, which can be either external (mechanical or ultrasonic push pulses) or intrinsic motion of the heart. LV filling following atrial kick (AK) in late ventricular diastole generates LV myocardial stretch that propagates with a speed related to myocardial stiffness. That is, myocardial stretch wave speed may be correlated with myocardial tissue stiffness. AK is the term for the increased force generated by the atria during contraction at the end of ventricular diastole before the ventricular systole when blood flows from the left atrium to the LV. Changes in myocardial stiffness have shown to be related to cardiac disease, particularly HFpEF. Therefore, a cardiac stiffness measurement tool could supplement differential diagnosis of HFpEF. Because intrinsic motions of the heart occur naturally, e.g., during or after AK, their generation does not require additional mechanical vibrators nor special transducers/excitations, hence often being referred to as "natural" waves. Compared to the most commonly used acoustic radiation force excitation in shear wave elastography, natural waves are characterized by higher displacements, potentially allowing them to travel and be tracked over larger distances. Measurement of myocardium stretch propagation speed following AK can assist in differential diagnosis of HFpEF, and may be more feasible than acoustic radiation force based techniques.

Engel Aaron J et al., "Cardiac atrial kick shear wave elastography with ultrafast diverging wave imaging: An in vivo pilot study", discloses a method of atrial kick examination using shear wave elastography.

Kvale Jaja F et al., "Detection of Tissue Fibrosis using Natural Mechanical Wave Velocity Estimation: Feasibility Study", discloses a method for mechanical wave velocity estimation for tissue fibrosis detection in the myocardium.

EP 2082688 A1 discloses a method of contrast agent enhanced medical diagnostic imaging.

### SUMMARY

The present disclosure describes systems and methods for providing user guidance to acquire optimal images containing a cardiac tissue of interest by showing an image quality score, provide automatic evaluation of frame scores within one or more cardiac phases of one or more cardiac cycles, and pass the phases when all frame scores within the phase is equal to or higher than a threshold value. The systems and methods disclosed herein may provide automatic post-processing of accepted cardiac phases and calculate the myocardial stretch wave speed (e.g., propagation speed) for each phase and exclude outliers. The systems and methods disclosed herein may provide a report showing all valid measurements and/or whisker plots of speed across all phases. In some applications, the systems and methods disclosed herein may improve accuracy and/or precision of differential HFpEF diagnosis. In some applications, the systems and methods disclosed herein may lead to more accurate and/or standardized myocardial stiffness measurements.

An ultrasound imaging system according to an example of the present disclosure may includean ultrasound transducer array configured to acquire ultrasound images of a heart, a display configured to display the ultrasound images, and a processor configured to calculate, for each of the ultrasound images, a score based, at least in part, on a portion of a cardiac tissue detected in each image, record image data of a region of interest (ROI) of the cardiac tissue for ultrasound images of the heart that have a score equal to or above a predetermined threshold value during at least one phase of a cardiac cycle of the heart, and for each recorded phase, calculate a propagation speed of the cardiac tissue from the recorded image data, wherein the propagation speed is calculated from image data of the recorded phases consisting only of images having a score equal to or above the predetermined threshold value, and provide the propagation speed on the display.

A method according to an example of the present disclosure may include acquiring ultrasound images of a heart, detecting a cardiac tissue in the ultrasound images, when the cardiac tissue is detected in the ultrasound images, analyzing the ultrasound images for a plurality of phases of one or more cardiac cycles to determine a quality score based, at least in part, on a percentage of the cardiac tissue in the ultrasound images, when the quality score for the ultrasound images for individual phases of the plurality of phases is equal or greater than a threshold value, calculating a propagation speed of the cardiac tissue for the individual phases, generating a report including the propagation speeds for the individual phases, and displaying the report on a display.

In accordance with an example of the present disclosure, a non-transitory computer-readable medium may contain instructions, that when executed, may cause an ultrasound imaging system to acquire ultrasound images of a heart, detect a cardiac tissue in the ultrasound images, when the cardiac tissue is detected in the ultrasound images, analyze the ultrasound images for a plurality of phases of one or more cardiac cycles to determine a quality score based, at least in part, on a percentage of the cardiac tissue in the ultrasound images, when the quality score for the ultrasound images for individual phases of the plurality of phases is equal or greater than a threshold value, calculate a propagation speed of the cardiac tissue for the individual phases, generate a report including the propagation speeds for the individual phases, and display the report on a display.

### BRIEF DESCRIPTION OF THE DRAWINGS

**FIG. 1** shows two examples of myocardium stretch speed measurement for a poor image acquisition and a good image acquisition.
**FIG. 2** is a block diagram of an ultrasound system in accordance with principles of the present disclosure.
**FIG. 3** is a block diagram illustrating an example processor in accordance with principles of the present disclosure.
**FIG. 4** is a block diagram of a process for training and deployment of a neural network in accordance with the principles of the present disclosure.
**FIG. 5A** is an example display according to an example of the present disclosure.
**FIG. 5B** is an example display according to the example of FIG. 5A.
**FIG. 5C** is an example display according to the example of FIGS. 5A and 5B.
**FIG. 6** illustrates generation of a curved anatomical m-mode image according to an example of the present disclosure.
**FIG. 7** is an example m-mode image of an atrial kick according to examples of the present disclosure.
**FIG. 8** is an example report according to examples of the present disclosure.
**FIG. 9** is a flow chart of a method according to examples of the present disclosure.

### DETAILED DESCRIPTION

The following description of certain embodiments is merely exemplary in nature and is in no way intended to limit the invention or its applications or uses. In the following detailed description of embodiments of the present systems and methods, reference is made to the accompanying drawings which form a part hereof, and which are shown by way of illustration specific embodiments in which the described systems and methods may be practiced. These embodiments are described in sufficient detail to enable those skilled in the art to practice presently disclosed systems and methods, and it is to be understood that other embodiments may be utilized and that structural and logical changes may be made without departing from the scope of the present system. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

Propagation speed (e.g., myocardial propagation speed) may be used as a measure of cardiac tissue stiffness and in turn a tool for differential diagnosis of heart failure with preserved ejection fraction (HFpEF). One method of measuring propagation speed is calculating a slope of an atrial kick (AK) from an m-mode image acquired across one or more cardiac phases and/or cycles. The m-mode image may track motion of points along a line through cardiac tissue over time (e.g., scan depth over time), for example, a line through the septum or a lateral wall of the heart. The line may be a scan line and/or an interpolation or other combination of multiple scan lines (e.g., where the line through the tissue is imaged by multiple scan lines). In some applications, the m-mode image may use grayscale or other color coding to indicate a velocity for points (e.g., depths) along the line for each time in the m-mode image. The slope of the AK in the m-mode image may be correlated with how quickly the motion of the AK propagates through the tissue along the line, that is, the slope may be correlated to the propagation speed.

In order to accurately measure the myocardium stretch propagation speed, it is important to include the tissue of interest (e.g., septum, lateral walls) in the field of view (e.g., in area or volume scanned by a transducer array), during the heart motion of the cardiac phase/cycle for tracking its displacement. It is also preferred to align a main (e.g., long) axis of the tracked tissue with the ultrasound beams as much as possible (e.g., the length of the septum parallel to the beam), in order to maximize displacement signal to noise ratio. This may be a significantly operator-dependent process in some applications. The view of the tissue and tissue coverage may vary depending on the user performing the scan or motion of the patient (e.g., breathing, coughing). Inter and intra operator variations can introduce measurement error and variability in evaluation of myocardium stretch propagation speed, leading to potential misdiagnosis. The inventors have found that cardiac view quality (e.g., adequate tissue coverage) is one of the primary reasons for failed measurements, followed by acoustic window quality (e.g., presence of clutter noise).

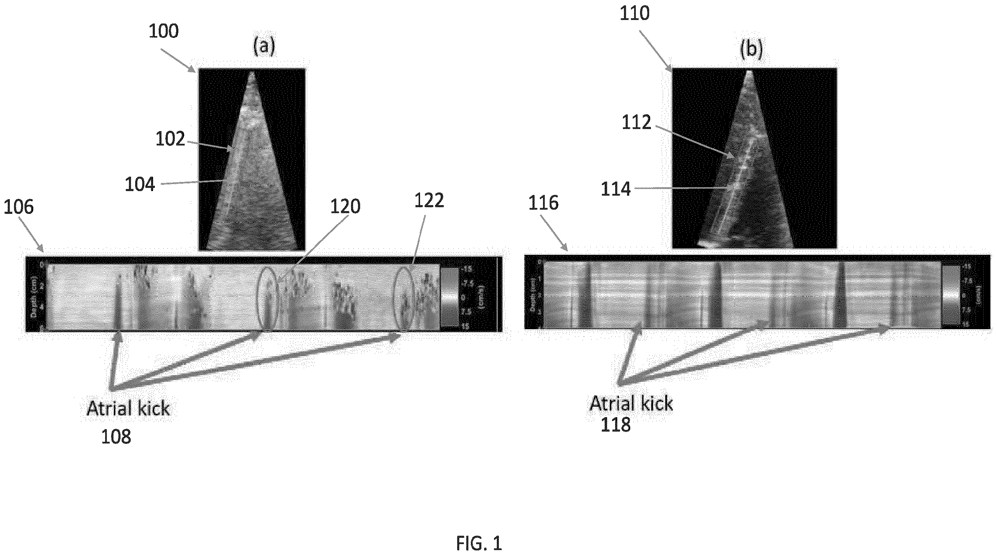

FIG. 1 shows two examples of myocardium stretch speed measurement for a poor image acquisition and a good image acquisition. Image 100 and image 110 are both cardiac ultrasound B-mode images including at least part of a septum. A portion of the septum is within a region of interest (ROI) 102 and indicated by line 104 in image 100 and the septum is within ROI 112 and line 114 in image 110. Below each image 100, 110 are corresponding m-mode images 106, 116. The m-mode images were acquired along lines 104, 114 over three cardiac cycles. Unlike image 110, in image 100, the septum is not completely within the image frame. As a result, the AK 108, particularly the second and third AK, indicated by circles 120 and 122, are noisy in comparison to the AK 118 of m-mode image 116. Thus, the measurements of myocardial propagation speed at the AK 108 of m-mode image 106 may be less reliable than the measurements at the AK 118 of m-mode image 116.

To reduce variations of myocardial propagation speed measurements, for example, due to poor image acquisition (e.g., only a portion of the tissue of interest located in the image, poor signal-to-noise ratio, poor angle between tissue wall and beam), standardized, semi-automatic, and/or automatic myocardium stretch propagation speed measurements may be desired. Such systems and/or methods may make a more accurate supplementary HFpEF diagnosis tool.

A semi-automatic technique for myocardial propagation speed measurements, which may improve not only the efficiency but also the success rate and reproducibility of a cardiac stiffness measurement workflow, is disclosed herein. As part of this semi-automatic technique, the quality of the frames associated with any given phase or phases of a cardiac cycle of the imaged tissue is evaluated during the cardiac ultrasound imaging. A cardiac cycle may be one heart beat, which includes one or more phases, such as a late diastolic phase. In some examples, a cardiac cycle may be measured, at least in part by an electrocardiography signal, for example, from a peak of a P-wave to a next P-wave, from a maximum peak of a QRS complex to a maximum peak of a next QRS complex. The image quality may be based on one or more metrics. For example, a metric may be based on cardiac tissue coverage in a B-mode image. The cardiac tissue may include a cardiac wall (e.g., septal wall, ventrical wall), valve (e.g., mitral valve), another portion of the heart (e.g., entire left ventrical), or combinations thereof. By coverage, it is meant what fraction or percentage of the cardiac wall is visible in the B-mode image. Other metrics for assessing the quality may include alignment of the wall with the beam, signal-to-noise ratio (SNR), and/or frame rate. In some examples, artificial intelligence (e.g., deep learning, machine learning) may be used to determine one or more of the metrics, for example, the wall coverage, position of the wall within the image, and/or alignment of the wall with the beam. Only phases where all images have acceptable quality (e.g., for which a quality score is equal to or exceeds a predetermined threshold) may be post-processed to calculate myocardial propagation speed measurements therefrom. Phases with images that do not meet the quality criteria are discarded in the myocardial propagation speed calculation process. In some examples, the phases may include an entire cardiac cycle. Myocardial propagation speed measurements may be obtained, for the select set of images, using any suitable technique. For example, rising slopes of the AK in corresponding m-mode images for the cardiac phases and/or cycles may be calculated. In some examples, post-processing may further include discarding outliers from the myocardial propagation speed measurements. The myocardial propagation speed measurements, obtained only for a select set of phases and/or cardiac cycles to enhance quality and repeatability, may be included in a report and/or used to generate whisker plots of propagation across the phases and/or cardiac cycles, which may be provided to the user (e.g., on a display).

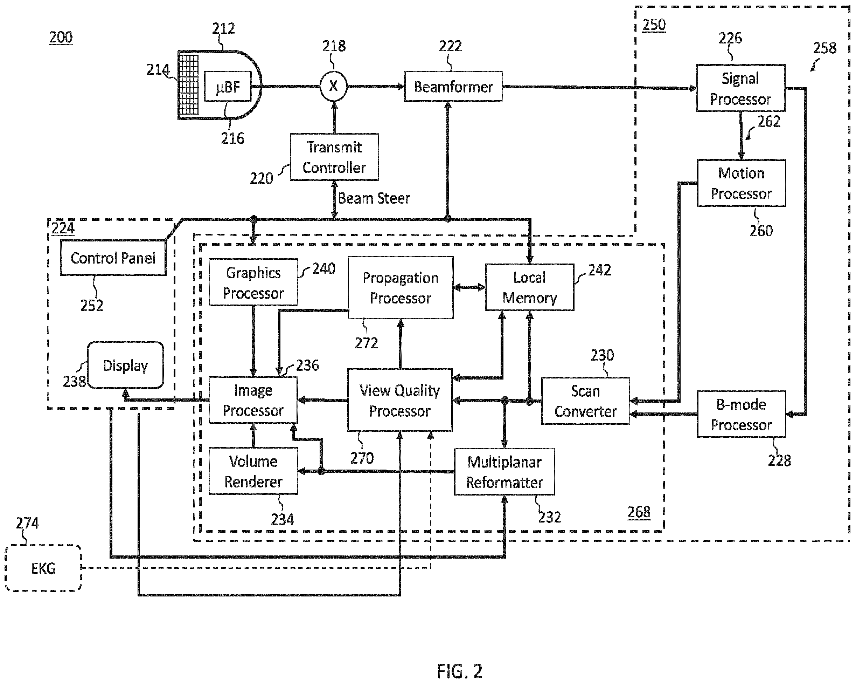

**FIG. 2** shows a block diagram of an ultrasound imaging system 200 constructed in accordance with the principles of the present disclosure. An ultrasound imaging system 200 according to the present disclosure may include a transducer array 214, which may be included in an ultrasound probe 212, for example an external probe or an internal probe such as an intravascular ultrasound (IVUS) catheter probe. In other embodiments, the transducer array 214 may be in the form of a flexible array configured to be conformally applied to a surface of subject to be imaged (e.g., patient). The transducer array 214 is configured to transmit ultrasound signals (e.g., beams, waves) and receive echoes responsive to the ultrasound signals. A variety of transducer arrays may be used, e.g., linear arrays, curved arrays, or phased arrays. The transducer array 214, for example, can include a two dimensional array (as shown) of transducer elements capable of scanning in both elevation and azimuth dimensions for 2D and/or 3D imaging. As is generally known, the axial direction is the direction normal to the face of the array (in the case of a curved array the axial directions fan out), the azimuthal direction is defined generally by the longitudinal dimension of the array, and the elevation direction is transverse to the azimuthal direction.

In some embodiments, the transducer array 214 may be coupled to a microbeamformer 216, which may be located in the ultrasound probe 212, and which may control the transmission and reception of signals by the transducer elements in the array 214. In some embodiments, the microbeamformer 216 may control the transmission and reception of signals by active elements in the array 214 (e.g., an active subset of elements of the array that define the active aperture at any given time).

In some embodiments, the microbeamformer 216 may be coupled, e.g., by a probe cable or wirelessly, to a transmit/receive (T/R) switch 218, which switches between transmission and reception and protects the main beamformer 222 from high energy transmit signals. In some embodiments, for example in portable ultrasound systems, the T/R switch 218 and other elements in the system can be included in the ultrasound probe 212 rather than in the ultrasound system base, which may house the image processing electronics. An ultrasound system base typically includes software and hardware components including circuitry for signal processing and image data generation as well as executable instructions for providing a user interface.

The transmission of ultrasonic signals from the transducer array 214 under control of the microbeamformer 216 is directed by the transmit controller 220, which may be coupled to the T/R switch 218 and a main beamformer 222. The transmit controller 220 may control the direction in which beams are steered. Beams may be steered straight ahead from (orthogonal to) the transducer array 214, or at different angles for a wider field of view. The transmit controller 220 may also be coupled to a user interface 224 and receive input from the user's operation of a user control. The user interface 224 may include one or more input devices such as a control panel 252, which may include one or more mechanical controls (e.g., buttons, encoders, etc.), touch sensitive controls (e.g., a trackpad, a touchscreen, or the like), and/or other known input devices.

In some embodiments, the partially beamformed signals produced by the microbeamformer 216 may be coupled to a main beamformer 222 where partially beamformed signals from individual patches of transducer elements may be combined into a fully beamformed signal. In some embodiments, microbeamformer 216 is omitted, and the transducer array 214 is under the control of the main beamformer 222 and main beamformer 222 performs all beamforming of signals. In embodiments with and without the microbeamformer 216, the beamformed signals of main beamformer 222 are coupled to processing circuitry 250, which may include one or more processors (e.g., a signal processor 226, a B-mode processor 228, a Doppler processor 260, and one or more image generation and processing components 268) configured to produce an ultrasound image from the beamformed signals (i.e., beamformed RF data).

The signal processor 226 may be configured to process the received beamformed RF data in various ways, such as bandpass filtering, decimation, I and Q component separation, and harmonic signal separation. The signal processor 126 may also perform additional signal enhancement such as speckle reduction, signal compounding, and noise elimination. The processed signals (also referred to as I and Q components or IQ signals) may be coupled to additional downstream signal processing circuits for image generation. The IQ signals may be coupled to a plurality of signal paths within the system, each of which may be associated with a specific arrangement of signal processing components suitable for generating different types of image data (e.g., B-mode image data, Doppler image data, m-mode image data).

For example, the system may include a B-mode signal path 258 which couples the signals from the signal processor 226 to a B-mode processor 228 for producing B-mode image data. The B-mode processor can employ amplitude detection for the imaging of structures in the body. In some examples, the B-mode image data may be processed to derive m-mode data.

The signals produced by the B-mode processor 228 may be coupled to a scan converter 230 and/or a multiplanar reformatter 232. The scan converter 230 may be configured to arrange the echo signals from the spatial relationship in which they were received to a desired image format. For instance, the scan converter 230 may arrange the echo signal into a two dimensional (2D) sector-shaped format, or a pyramidal or otherwise shaped three dimensional (3D) format. The multiplanar reformatter 232 can convert echoes which are received from points in a common plane in a volumetric region of the body into an ultrasonic image (e.g., a B-mode image) of that plane, for example as described in U.S. Pat. No. 6,443,896 (Detmer). The scan converter 230 and multiplanar reformatter 232 may be implemented as one or more processors in some embodiments.

A volume renderer 234 may generate an image (also referred to as a projection, render, or rendering) of the 3D dataset as viewed from a given reference point, e.g., as described in U.S. Pat. No. 6,530,885 (Entrekin et al.). The volume renderer 234 may be implemented as one or more processors in some embodiments. The volume renderer 234 may generate a render, such as a positive render or a negative render, by any known or future known technique such as surface rendering and maximum intensity rendering.

In some embodiments, the system may include a motion signal path 262 which couples the output from the signal processor 226 to a motion processor 260. The motion processor 260 may be configured to estimate the Doppler shift and generate Doppler image data based on motion (e.g., blood flow). The Doppler image data may include color data which is then overlaid with B-mode (e.g., grayscale) image data for display. The motion processor 260 may filter out unwanted signals (e.g., noise or clutter associated with non-moving tissue), for example using a wall filter. The motion processor 260 may be further configured to estimate velocity and power in accordance with known techniques. For example, the motion processor 260 may include a Doppler estimator such as an auto-correlator, in which velocity (Doppler frequency) estimation is based on the argument of the lag-one autocorrelation function and Doppler power estimation is based on the magnitude of the lag-zero autocorrelation function. Motion can also be estimated by known phase-domain (for example, parametric frequency estimators such as MUSIC, ESPRIT, etc.) or time-domain (for example, cross-correlation) signal processing techniques. Other estimators related to the temporal or spatial distributions of velocity such as estimators of acceleration or temporal and/or spatial velocity derivatives can be used instead of or in addition to velocity estimators. In some embodiments, the velocity and power estimates may undergo further threshold detection to further reduce noise, as well as segmentation and post-processing such as filling and smoothing. The velocity and power estimates may then be mapped to a desired range of display colors in accordance with a color map. The color data, also referred to as Doppler image data, may then be coupled to the scan converter 230, where the Doppler image data may be converted to the desired image format and overlaid on the B-mode image of the tissue structure to form a color Doppler or a power Doppler image.

In some examples, the motion processor 260 may generate additional image data based on motion that may or may not rely on Doppler shifts, such as m-mode data. Thus, m-mode data need not always be generated from the B-mode data. The m-mode data may be provided from the motion processor 260 to the scan converter 230.

According to principles of the present disclosure, output from the scan converter 230, such as B-mode images, Doppler images, and/or m-mode images, may be provided to a view quality processor 270. The view quality processor 270 may analyze the B-mode ultrasound images to determine a quality of the images based on one or more metrics. For example, the quality processor 270 may analyze the ultrasound images to determine whether a cardiac tissue, or a portion thereof, is within the images, whether the cardiac tissue is aligned with the beam of the transducer array 214, whether the SNR at the cardiac tissue is the same or better than the SNR of surrounding tissue in the image, and/or whether the frame rate is sufficient. Other suitable metrics may also be used to evaluate quality of the images (e.g., artifact detection, beam distortion detection).

In some embodiments, the view quality processor 270 may utilize a neural network, for example a deep neural network (DNN), a convolutional neural network (CNN), a recurrent neural network (RNN), an autoencoder neural network, or the like, to recognize a cardiac tissue, such as the septum. Other example networks include you only look once (YOLO) networks, regional-CNN (R-CNN), and Faster R-CNN. The neural network may be implemented in hardware (e.g., neurons are represented by physical components) and/or software (e.g., neurons and pathways implemented in a software application) components. The neural network implemented according to the present disclosure may use a variety of topologies and learning algorithms for training the neural network to produce the desired output. For example, a software-based neural network may be implemented using a processor (e.g., single or multi-core CPU, a single GPU or GPU cluster, or multiple processors arranged for parallel-processing) configured to execute instructions, which may be stored in computer readable medium, and which when executed cause the processor to perform a trained algorithm for determining whether the cardiac wall is within a B-mode image and/or what portion of the cardiac tissue is within the B-mode image. In some examples, the algorithm may be trained to determine an orientation of the cardiac tissue in relation to an ultrasound beam from the transducer array 214.

In various embodiments, the neural network(s) may be trained using any of a variety of currently known or later developed learning techniques to obtain a neural network (e.g., a trained algorithm or hardware-based system of nodes) that is configured to analyze input data in the form of ultrasound images, measurements, and/or statistics and determine whether the cardiac wall and/or what portion of the cardiac tissue is within the B-mode image. In some embodiments, the neural network may be statically trained. That is, the neural network may be trained with a data set and deployed on the view quality processor 270. In some embodiments, the neural network may be dynamically trained. In these embodiments, the neural network may be trained with an initial data set and deployed on the view quality processor 270. However, the neural network may continue to train and be modified based on ultrasound images acquired by the system 200 after deployment of the neural network on the view quality processor 270.

In other embodiments, the view quality processor 270 may not include a neural network. In other embodiments, the view recognition processor 270 may be implemented using any other suitable machine learning and/or image processing technique, such as image segmentation, histogram analysis, edge detection, or other shape or object recognition techniques. In some embodiments, the view quality processor 270 may implement a neural network in combination with other machine learning and/or image processing methods to determine whether the cardiac tissue is located within the B-mode image.

In some examples, once at least a portion of the cardiac tissue is determined to be in the B-mode image, the coverage of the cardiac tissue may be deemed sufficient when at least seventy, eighty, ninety, or ninety-five percent of the cardiac tissue is within the B-mode image. For example, the coverage may be deemed sufficient when at least ninety percent of the septum is within the B-mode image.

In some embodiments, image segmentation, histogram analysis, speckle analysis, and/or other imaging techniques may be used by the view quality processor 270 to determine the signal-to-noise ratio and/or the alignment of the cardiac tissue with the ultrasound beam. In some examples, the signal-to-noise ratio may be deemed to be sufficient if the signal-to-noise ratio of the cardiac tissue is equal to or greater than the signal-to-noise ratio of other surrounding tissue in the B-mode image. In some examples, the alignment of the cardiac tissue may be sufficient when the cardiac wall is at an angle less than ninety degrees (e.g., orthogonal) relative to the beam. In some examples, the alignment of the cardiac tissue may be sufficient when the cardiac tissue is at an angle less than ten, twenty, thirty, forty, fifty, or sixty degrees relative to the beam.

In some embodiments, the frame rate may be determined based on information from other components of the system 200 (e.g., control panel 252, transmit controller 220, scan converter 230), a rate at which images are received from the scan converter 230, and/or information included with the ultrasound images (e.g., time stamps). In some embodiments, the frame rate may be deemed sufficient when the frame rate is equal to or greater than 100, 200, or 300 frames per second.

In some examples, the view quality processor 270 may generate a score (also referred to as a quality score) based on the one or more metrics as a measure of image quality. In some examples, the score may have a value equal to or between zero and one hundred. In some examples, the score may have a value equal to or between zero and one. In some embodiments, the score may be a weighted sum of multiple metrics. The metrics may not be weighted equally in some embodiments. For example, whether or not a sufficient portion of the cardiac tissue is within the image may be weighted higher than the SNR of the image. In some embodiments, the view quality processor 270 may compare the score to a threshold value. The view quality processor 270 may determine that an image of sufficient quality to be included in a myocardial propagation speed measurement calculation if the score of the image is equal to or greater than a threshold value (e.g., equal to or over 90, equal to or over 0.90). In some examples, the view quality processor 270 may calculate a score for every image of a given phase and/or cardiac cycle to determine whether a myocardial propagation speed measurement should be obtained from that phase and/or cardiac cycle. In some embodiments, a myocardial propagation speed measurement is obtained only from phases and/or cardiac cycles for which all images of the phase and/or cardiac cycle meet the quality criteria (e.g., have a score equal to or above the threshold value). This may improve reliability and/or reproducibility of myocardial propagation speed measurements as phases and/or cardiac cycles with poor images (e.g., the septum falls out of or partially out of the field of view during the AK) are not used to generate the measurements. In some embodiments, the view quality processor 270 may determine which images belong to a particular phase and/or cardiac cycle based on an analysis of the images. In some embodiments, the view quality processor 270 may determine which images belong to a particular phase and/or cardiac cycle based on an electrocardiography (EKG) signal provided by an electrocardiography sensor 274 coupled to the system 200.

In some embodiments, the view quality processor 270 may provide the score to an image processor 236, which may provide the score on a display 238. In some embodiments, the view quality processor 270 may initially determine the score without regard to phases or cardiac cycles. Once the score equals or exceeds the threshold value, the user interface 224 may allow a user to start acquiring images across one or more phases and/or cardiac cycles. This may alert the user to when a satisfactory position and orientation of the probe 212 for myocardial propagation speed calculations have been obtained. In some examples, a number of phases and/or cardiac cycles may be selected by the user via a user input provided via the user interface 224. The view quality processor 270 may begin determining the quality of images within each phase and/or cardiac cycle. In some embodiments, when the quality of all the images of a phase and/or cardiac cycle is sufficient, the view quality processor 270 may provide the B-mode, Doppler, and/or m-mode images to a propagation processor 272. In some embodiments, the m-mode image provided by the view quality processor 270 may be based, at least in part, on analysis of the cardiac tissue. For example, the line for which the m-mode image is generated may be along the cardiac tissue. In another example, the view quality processor 270 may segment the image to automatically determine a region of interest (ROI) including the cardiac tisuse and the line for the m-mode image may be taken along a center of the ROI. In some embodiments, the view quality processor 270 may continue determining the quality of the images until a desired number of phases and/or cardiac cycles with sufficient image quality are acquired. Thus, the user need not trigger additional phase and/or cardiac cycle acquisitions if a consecutive number of phase and/or cardiac cycles do not have sufficient image quality.

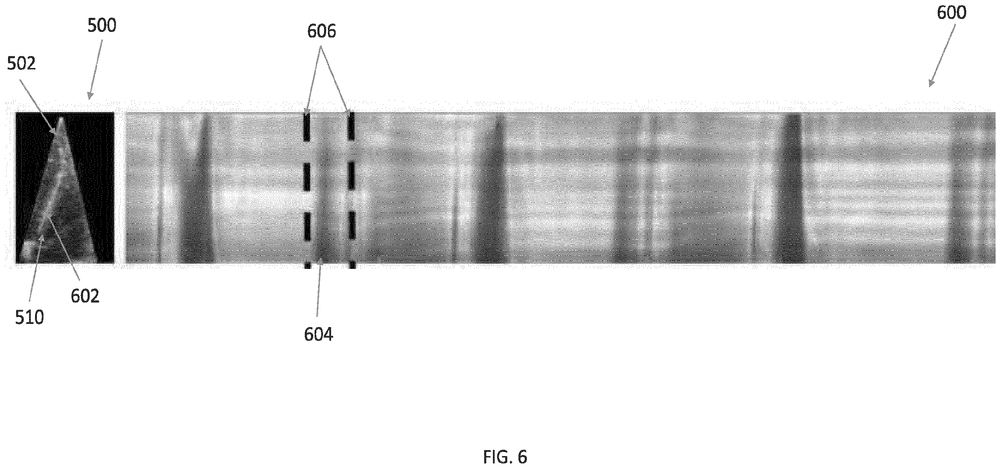

As will be described in more detail with reference to FIGS. 6-8, the propagation processor 272 may analyze the ultrasound images to calculate a propagation speed, that is, movement of the tissue of the cardiac tissue. For example, the propagation processor 272 may find a slope of the AK in the m-mode image. In some embodiments, the propagation processor 272 may receive data from the view quality processor 270 and/or the EKG sensor 274 to determine the location of the AK in the m-mode image. The propagation processor 272 may calculate the propagation speed for each phase and/or cardiac cycle. In some embodiments, the number of phases and/or cardiac cycles analyzed by the propagation processor 272 may be determined by a user. In some embodiments, the propagation processor 272 may generate a report. The report may include some or all of the propagation speeds calculated. In some embodiments, outlier values for propagation speeds may be excluded from the report. For example, values for propagation speeds greater or less than 20% of the mean speed across all phases and/or cardiac cycles may be removed. In some embodiments, in addition to the numerical values of the propagation speeds, the report may include a whisker plot to provide a visual indication of the variability of the calculated speeds. In some embodiments, the report may be provided to the image processor 236.

In addition to the score and report provided by the view quality processor 270 and propagation processor 272, respectively, output (e.g., B-mode images, Doppler images) from the scan converter 230, the multiplanar reformatter 232, and/or the volume renderer 234 may be coupled to an image processor 236 for further enhancement, buffering and temporary storage before being displayed on an image display 238. Although output from the scan converter 230 is shown as provided to the image processor 236 via the view quality processor 270, in some embodiments, the output of the scan converter 230 may be provided directly to the image processor 236. A graphics processor 240 may generate graphic overlays for display with the images. These graphic overlays can contain, e.g., standard identifying information such as patient name, date and time of the image, imaging parameters, and the like. For these purposes, the graphics processor may be configured to receive input from the user interface 224, such as a typed patient name or other annotations. In some embodiments, the view quality processor 270 and/or propagation processor 272 may be coupled to the graphics processor 240 in addition to or instead of the image processor 236. The user interface 244 can also be coupled to the multiplanar reformatter 232 for selection and control of a display of multiple multiplanar reformatted (MPR) images.

The system 200 may include local memory 242. Local memory 242 may be implemented as any suitable non-transitory computer readable medium (e.g., flash drive, disk drive). Local memory 242 may store data generated by the system 200 including ultrasound images, executable instructions, myocardial propagation speeds, training data sets, or any other information necessary for the operation of the system 200.

As mentioned previously system 200 includes user interface 224. User interface 224 may include display 238 and control panel 252. The display 238 may include a display device implemented using a variety of known display technologies, such as LCD, LED, OLED, or plasma display technology. In some embodiments, display 238 may comprise multiple displays. The control panel 252 may be configured to receive user inputs (e.g., exam type, threshold confidence score, number of phases or cardiac cycles). The control panel 252 may include one or more hard controls (e.g., buttons, knobs, dials, encoders, mouse, trackball, or others). In some embodiments, the control panel 252 may additionally or alternatively include soft controls (e.g., GUI control elements or simply, GUI controls) provided on a touch sensitive display. In some embodiments, display 238 may be a touch sensitive display that includes one or more soft controls of the control panel 252.

In some embodiments, various components shown in FIG. 2 may be combined. For instance, image processor 236 and graphics processor 240 may be implemented as a single processor. In another example, the view quality processor 270 and the propagation processor 272 may be implemented as a single processor. In some embodiments, various components shown in FIG. 2 may be implemented as separate components. For example, signal processor 226 may be implemented as separate signal processors for each imaging mode (e.g., B-mode, Doppler). In some embodiments, one or more of the various processors shown in FIG. 2 may be implemented by general purpose processors and/or microprocessors configured to perform the specified tasks. In some embodiments, one or more of the various processors may be implemented as application specific circuits. In some embodiments, one or more of the various processors (e.g., image processor 236) may be implemented with one or more graphical processing units (GPU).

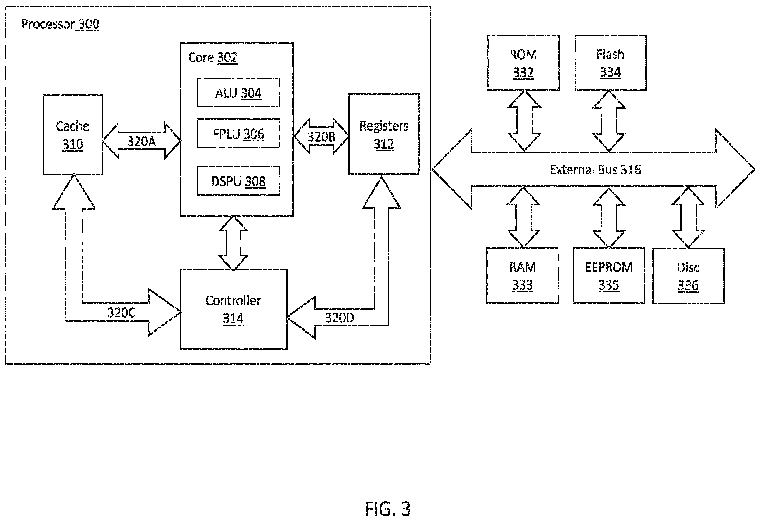

FIG. 3 is a block diagram illustrating an example processor 300 according to principles of the present disclosure. Processor 300 may be used to implement one or more processors and/or controllers described herein, for example, image processor 236 shown in FIG. 2 and/or any other processor or controller shown in FIG. 2. Processor 300 may be any suitable processor type including, but not limited to, a microprocessor, a microcontroller, a digital signal processor (DSP), a field programmable gate array (FPGA) where the FPGA has been programmed to form a processor, a graphical processing unit (GPU), an application specific circuit (ASIC) where the ASIC has been designed to form a processor, or a combination thereof.

The processor 300 may include one or more cores 302. The core 302 may include one or more arithmetic logic units (ALU) 304. In some embodiments, the core 302 may include a floating point logic unit (FPLU) 306 and/or a digital signal processing unit (DSPU) 308 in addition to or instead of the ALU 304.

The processor 300 may include one or more registers 312 communicatively coupled to the core 302. The registers 312 may be implemented using dedicated logic gate circuits (e.g., flip-flops) and/or any memory technology. In some embodiments, the registers 312 may be implemented using static memory. The register may provide data, instructions and addresses to the core 302.

In some embodiments, processor 300 may include one or more levels of cache memory 310 communicatively coupled to the core 302. The cache memory 310 may provide computer-readable instructions to the core 302 for execution. The cache memory 310 may provide data for processing by the core 302. In some embodiments, the computer-readable instructions may have been provided to the cache memory 310 by a local memory, for example, local memory attached to the external bus 316. The cache memory 310 may be implemented with any suitable cache memory type, for example, metal-oxide semiconductor (MOS) memory such as static random access memory (SRAM), dynamic random access memory (DRAM), and/or any other suitable memory technology.

The processor 300 may include a controller 314, which may control input to the processor 300 from other processors and/or components included in a system (e.g., control panel 252 and scan converter 230 shown in FIG. 2) and/or outputs from the processor 300 to other processors and/or components included in the system (e.g., display 238 and volume renderer 234 shown in FIG. 2). Controller 314 may control the data paths in the ALU 304, FPLU 306 and/or DSPU 308. Controller 314 may be implemented as one or more state machines, data paths and/or dedicated control logic. The gates of controller 314 may be implemented as standalone gates, FPGA, ASIC or any other suitable technology.

The registers 312 and the cache memory 310 may communicate with controller 314 and core 302 via internal connections 320A, 320B, 320C and 320D. Internal connections may implemented as a bus, multiplexor, crossbar switch, and/or any other suitable connection technology.

Inputs and outputs for the processor 300 may be provided via a bus 316, which may include one or more conductive lines. The bus 316 may be communicatively coupled to one or more components of processor 300, for example, the controller 314, cache 310, and/or register 312. The bus 316 may be coupled to one or more components of the system, such as display 238 and control panel 252 mentioned previously.

The bus 316 may be coupled to one or more external memories. The external memories may include Read Only Memory (ROM) 332. ROM 332 may be a masked ROM, Electronically Programmable Read Only Memory (EPROM) or any other suitable technology. The external memory may include Random Access Memory (RAM) 333. RAM 333 may be a static RAM, battery backed up static RAM, Dynamic RAM (DRAM) or any other suitable technology. The external memory may include Electrically Erasable Programmable Read Only Memory (EEPROM) 335. The external memory may include Flash memory 334. The external memory may include a magnetic storage device such as disc 336. In some embodiments, the external memories may be included in a system, such as ultrasound imaging system 200 shown in Fig. 2, for example local memory 242.

In some embodiments, the system 200 can be configured to implement a neural network or other artificial intelligence technique included in the view quality processor 270. For example, the view quality processor 270 may include a CNN, to determine whether a cardiac tissue is present in an image, a portion (e.g., percentage) of the cardiac tissue included in the image, an angle of the cardiac tissue relative to the ultrasound beam, and/or a center of the cardiac tissue (e.g., for selecting a line for an m-mode image). The neural network may be trained to perform these functions with imaging data such as image frames where the cardiac tissue is labeled as present, the portion of the cardiac tissue is labeled, the angle of the cardiac tissue relative to the ultrasound beam is labeled, and/or a center of the cardiac tissue is labeled.

In some embodiments, a neural network training algorithm associated with the neural network can be presented with thousands or even millions of training data sets in order to train the neural network. In various embodiments, the number of ultrasound images used to train the neural network(s) may range from about 50,000 to 200,000 or more. The number of images used to train the network(s) may be increased if higher numbers of different items of interest are to be identified, or to accommodate a greater variety of patient variation, e.g., hearts with and without implants, artificial valves, etc. The number of training images may differ for different items of interest or features thereof, and may depend on variability in the appearance of certain features. For example, pediatric patients typically have a greater range of variability across age ranges than adults. Training the network(s) to assess the presence of items of interest associated with features for which population-wide variability is high may necessitate a greater volume of training images.

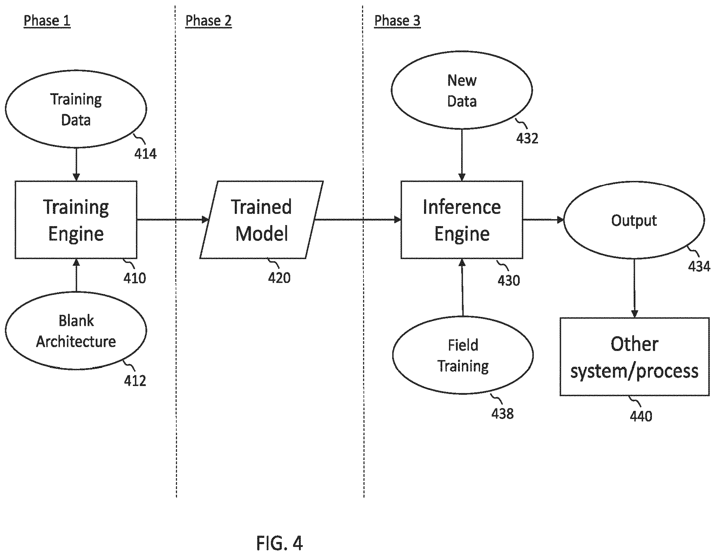

**FIG. 4** shows a block diagram of a process for training and deployment of a neural network in accordance with the principles of the present disclosure. The process shown in FIG. 4 may be used to train a neural network included in view quality processor 270. The left hand side of FIG. 4, phase 1, illustrates the training of a neural network. To train the neural network, training sets which include multiple instances of input arrays and output classifications may be presented to the training algorithm(s) of the neural network(s) (e.g., AlexNet training algorithm, as described by Krizhevsky, A., Sutskever, I. and Hinton, G. E. "ImageNet Classification with Deep Convolutional Neural Networks, " NIPS 2012 or its descendants). Training may involve the selection of a starting network architecture 412 and the preparation of training data 414. The starting network architecture 412 may be a blank architecture (e.g., an architecture with defined layers and arrangement of nodes but without any previously trained weights) or a partially trained network, such as the inception networks, which may then be further tailored for classification of ultrasound images. The starting architecture 412 (e.g., blank weights) and training data 414 are provided to a training engine 410 for training the model. Upon sufficient number of iterations (e.g., when the model performs consistently within an acceptable error), the model 420 is said to be trained and ready for deployment, which is illustrated in the middle of FIG. 4, phase 2. The right hand side of FIG. 4, or phase 3, the trained model 420 is applied (via inference engine 430) for analysis of new data 432, which is data that has not been presented to the model during the initial training (in phase 1). For example, the new data 432 may include unknown images such as live ultrasound images acquired during a scan of a patient (e.g., cardiac images during an echocardiography exam). The trained model 420 implemented via engine 430 is used to classify the unknown images in accordance with the training of the model 420 to provide an output 434 (e.g., cardiac tissue present, percentage of cardiac tissue present, angle of cardiac tissue relative to ultrasound beam). The output 434 may then be used by the system for subsequent processes 440 (e.g., calculating a score for the image, selecting a line for generating an m-mode image).

In embodiments where the neural network is dynamically trained, the engine 430 may receive field training 438. The engine 430 may continue to train and be modified based on data acquired after deployment of the engine 430. The field training 438 may be based, at least in part, on new data 432 in some embodiments.

In the embodiments where the trained model 420 is used to implement a neural network of the view quality processor 270, the starting architecture may be that of a convolutional neural network, or a deep convolutional neural network, which may be trained to perform object recognition, image segmentation, image comparison, or any combinations thereof. With the increasing volume of stored medical image data, the availability of high-quality clinical images is increasing, which may be leveraged to train a neural network to learn the probability of a given image frame containing a cardiac tissue (e.g., confidence score). The training data 414 may include multiple (hundreds, often thousands or even more) annotated/labeled images, also referred to as training images. It will be understood that the training image need not include a full image produced by an imagining system (e.g., representative of the full field of view of the probe) but may include patches or portions of images of the labeled item of interest.

In various embodiments, the trained neural network may be implemented, at least in part, in a computer-readable medium comprising executable instructions executed by a processor, e.g., view quality processor 270. In some embodiments in which an ultrasound imaging system implements a trained neural network, deployed on the ultrasound system to detect a cardiac tissue in acquired images, the quality score used by the ultrasound system for selecting and excluding images, cardiac phases, and/or cardiac cycles from the myocardial propagation speed measurements may correspond to or be based, at least in part, on the probability output (e.g., the confidence score) generated by the neural network as part of the image recognition process.

Examples of operation of an ultrasound imaging system for automatically and/or semiautomatically measuring propagation speed will now be described. The operations described herein may be performed by ultrasound imaging system 200 in some embodiments.

In some embodiments, the user may select a Cardiac Stiffness Measurmeent (CSM) hard or soft control on a user interface (e.g., user interface 224) responsive to which a CSM application for performing image quality analysis and calculating myocardial propagation speed values is executed by the ultrasound system. A user interface of the CSM application may include an interface element configured to enable the user to specify the number of cardiac cycles to be acquired for calculating propagation speed measurements. The user interface element may be a soft control of the user interface (e.g., a GUI on a touch screen) or one or more hard controls (e.g., button(s), a touch pad, keyboard) dedicated or re-configured to receive user inputs associated with operation of the CSM application. Although the example described herein uses cardiac cycles, in other embodiments, one or more cardiac phases may be designated instead of or in addition to cardiac cycles.

As the user moves an ultrasound transducer (e.g., ultrasound transducer 214), a quality score, which may be referred to in some examples as a "View Quality Score," may be calculated for every new image, for example, by view quality processor 270, in some embodiments in real-time, as the transducer acquires each new image. The score may be displayed on a display, such as display 238, next to a B-mode image at the same frame rate of B-mode imaging. In some embodiments, the score may be displayed numerically (e.g., on or next to the associated image) or may be displayed relative to a range or scale, such as in the case of a colorbar where the score is visually displayed as a bar or other marker, which can move dynamically along the colorbar to indicate where in the quality range the score of the current image falls. In some embodiments, the display of the score may be a combination of a quantitative (e.g., a numerical score) and a relative representation (e.g., in relation to the range of scores) may be provided, for example by also displaying the numerical score next to (e.g. adjacent, above or below) the bar and dynamically updating the numerical score as the bar moves along the ranged (e.g., the colorbar).

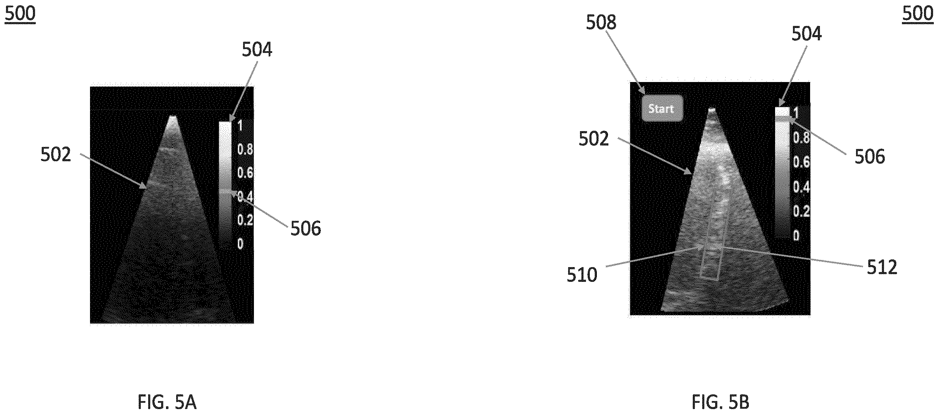

**FIG. 5A** is an example display according to an example of the present disclosure. The display 500 provides the B-mode image 502 and a visual representation of the score. In the example shown, a colorbar 504 representation is used to visualize the score with increasing view quality. The horizontal bar 506 on the colorbar 504 may move up and down to give user real time or near-real time feedback on image quality.

**FIG. 5B** is an example display according to the example shown in FIG. 5A. Once a view in the B-mode image 502 has a score that meets or exceeds a threshold value (e.g., 0.9 in the example shown), a "start" button 508 may appear. In a semi-automated mode, the user may click the start button 508 to begin acquiring images for calculating the myocardial propagation speed measurements. In other modes of the CSM application, the ultrasound system may acquire and process cardiac images for calculating the myocardial propagation speed measurements automatically when the probe is positioned to capture the appropriate view (e.g., a view substantially fully containing the tissue of interest, such as substantially fully including the septum or a particular wall of the heart).

Still referring to FIG. 5B, the ultrasound imaging system may automatically select a region of interest (ROI) 510 within the B-mode image 502. In some embodiments, selection of the ROI may be activated simultaneously (or near simultaneously) with the view score calculation. In some embodiments, selection of the ROI may also be performed by the view quality processor 270. Once the B-mode image 502 has a score that meets or exceeds the threshold value, segmentation and/or cardiac wall 512 identification may be performed to select the ROI 510. In the example shown, the cardiac tissue is the cardiac wall 512, which is the ventricular septum. However, in other examples, the cardiac tissue may include additional or other portions of the heart.

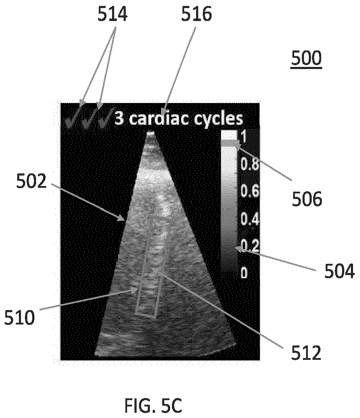

After user actuates the "start" button 508, if a view with an acceptable score is maintained during at least one full cardiac cycle, the ultrasound imaging system may save the image data (e.g., B-mode, Doppler, m-mode) separately for individual cardiac cycles. Optionally, the ultrasound imaging system may provide a notification sound (e.g., ding or beep) and/or visually shows the number of successfully acquired cardiac cycles after they are captured either symbolically (e.g., checkmarks 514 as shown in **FIG. 5C**) and/or as text 516. The user may have an option to disable the notification sound or display. The ultrasound imaging system repeats the process until image data for a desired number of cardiac cycles is successfully captured (e.g., all images for each cardiac cycle have scores equal or above the threshold value). When the notification is disabled, the CSM application may transition to the myocardial propagation speed determination step automatically upon acquiring the desired number (e.g., one or more) of cardiac cycles that meet the quality criteria.

Once the desired number of cardiac cycles have been captured, the image data may be processed to calculate myocardial propagation speeds. The post-acquisition processing may be performed by the view quality processor 270 and/or propagation processor 272. For example, as shown in **FIG. 6****,** the view quality processor 270 or a user may select a line 602 through the center of the ROI 510 and/or through the cardiac wall (e.g., interventricular septum mid-line). Motion fields for only for individual cardiac cycles that are included in the image data used for the propagation speed measurements may be projected and unfolded to provide a curved anatomical m-mode (CAMM) image data 600 of tissue velocity. The processor (e.g., propagation processor 272) identifies an atrial kick (AK) 604 (indicated in this example between dashed lines 606) of the cardiac cycle from which the myocardial propagation speed may be calculated.

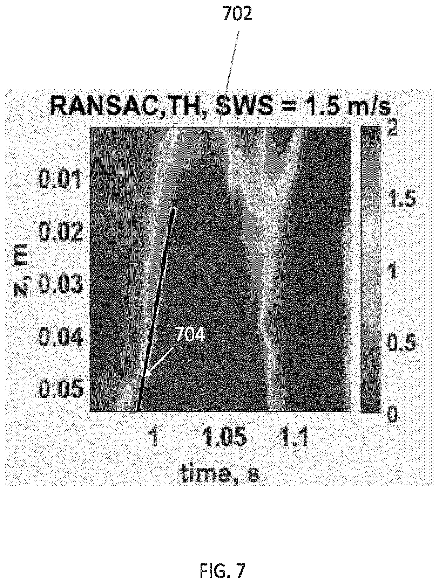

In some embodiments, the rising slope of the arterial kick (AK) may be used to calculate the myocardial propagation speed. **FIG. 7** is an example m-mode image of an AK according to examples of the present disclosure. The propagation processor 272 determines the rising slope 704 of the AK 702, which may indicate the myocardial propagation speed. In some embodiments, the propagation processor 272 may use a random sample consensus (RANSAC) algorithm to calculate the slope 704. RANSAC is an iterative linear fitting method that may be suitable for interpreting data including significant numbers of outliers. In other examples, a different technique for determining the slope 704 may be used. Each of the recorded image data 600 for each cardiac cycle that meets the quality criteria may be similarly processed to determine myocardial propagation speeds for the desired number of cardiac cycles, and the individual measurements from each cycle may be provided to the user. In some embodiments, a single (e.g., an average, mean, or median) measurement based on the individual measurements from the processed cardiac cycles may be provided. Optionally, the measurements may be further evaluated and cardiac cycles are calculated, outliers may be excluded (e.g., values above or below 20% of the mean speed) from the output (e.g., a report) provided to the user.

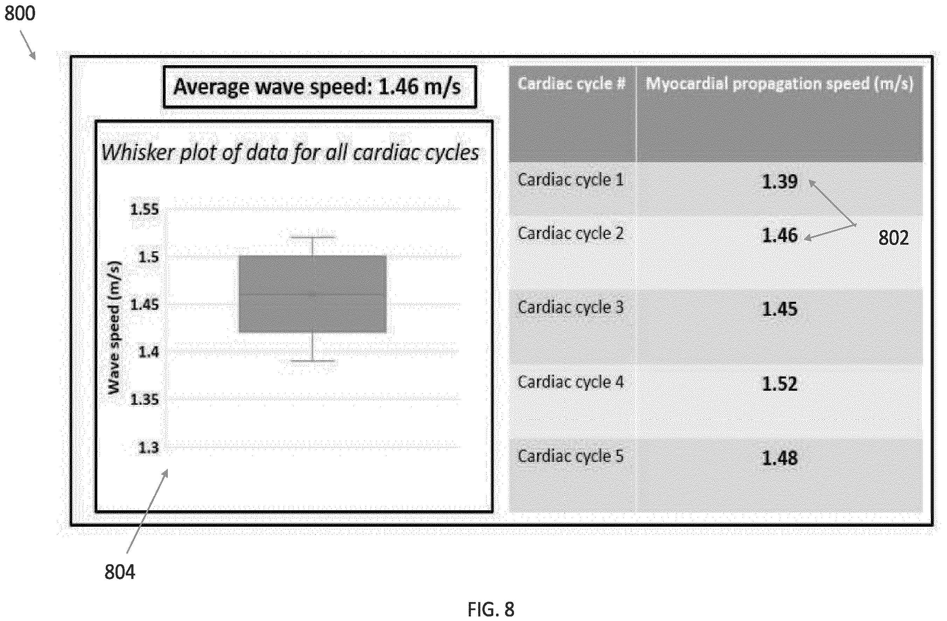

**FIG. 8** is an example report according to examples of the present disclosure. The myocardial propagation speeds may be provided to the user as a report 800 by the propagation processor 272, for example, via the display. In some examples, such as the one shown, the report 800 may include the calculated myocardial propagation speeds 802 for individual cardiac cycles. In some examples, the report 800 may include a whiskers plot 804 in addition to or instead of the individually displayed myocardial propagation speeds 802. As mentioned above, outliers may be omitted from the report 800.

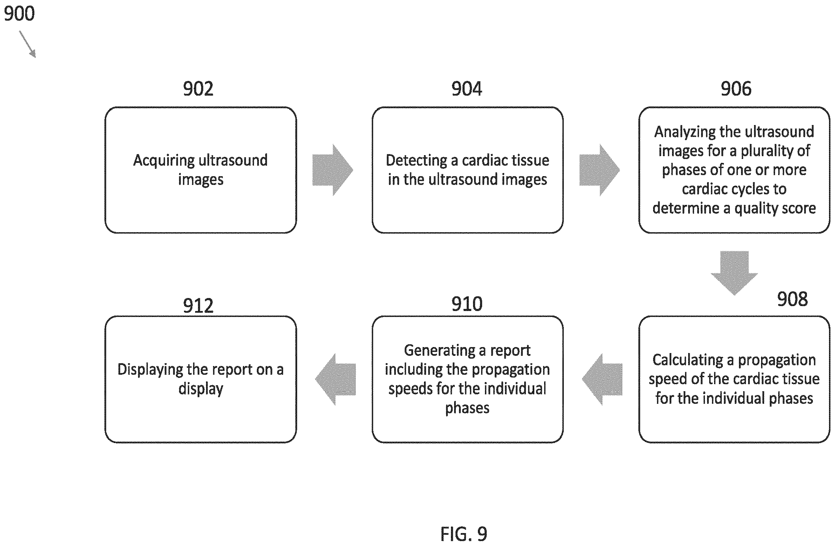

**FIG. 9** is a flow chart of a method according to examples of the present disclosure. The method 900 may be performed by the ultrasound imaging system 200 in some embodiments. At block 902, "acquiring ultrasound images" may be performed. The ultrasound images may be acquired by an ultrasound probe, such as ultrasound probe 212. Focused, plane, and/or diverging beams may be used to acquire the ultrasound images. At block 904, "detecting a cardiac tissue in the ultrasound images" may be performed. In some embodiments, the detecting may be performed by a processor, such as view quality processor 270. In some embodiments, the processor may implement an AI technique, such as a neural network, for determining when the cardiac tissue is included in the ultrasound images.

When the cardiac tissue is included in the ultrasound images, at block 906, "analyzing the ultrasound images for a plurality of phases of one or more cardiac cycles to determine a quality score" may be performed by the processor. In some embodiments, the quality score may be based on at least one of a presence of the cardiac tissue in the ultrasound images, a percentage of the cardiac tissue in the ultrasound images, an angle of the cardiac tissue relative to an ultrasound beam, a signal-to-noise ratio at the cardiac tissue, or a frame rate of the ultrasound images.

When the quality score for the ultrasound images for individual phases and/or cardiac cycles of the plurality of phases and/or cardiac cycles is equal or greater than a threshold value, at block 908, "calculating a propagation speed of the cardiac tissue for the individual phases" may be performed. In some embodiments, the calculating may be performed by the processor or another processor, such as propagation processor 272. In some embodiments, calculating the propagation speed may include calculating a slope of an atrial kick in an m-mode image, wherein the m-mode image is acquired along a line through the cardiac tissue. In some embodiments, the cardiac tissue may be segmented from the ultrasound images and placing the line through the cardiac tissue. In some embodiments, the segmenting may be performed by the processor. In some embodiments, a region of interest may be generated in the ultrasound images including the cardiac tissue and placing the line through a center of the region of interest. In some embodiments, the generation of the region of interest may be performed by the processor. In some embodiments, a user input may be received (e.g., via user interface 224) indicating a desired number of phases and/or cardiac cycles, wherein a number of the individual phases and/or cardiac cycles for which the myocardial propagation speed is calculated is equal to the desired number of phases and/or cardiac cycles.

At block 910, "generating a report including the propagation speeds for the individual phases" may be performed. In some embodiments, generating the report may be performed by the processor. In some embodiments, the processor may determine a mean value for the propagation speeds and omit outlier propagation speeds from the report, wherein outlier propagation speeds have values greater or less than twenty percent of the mean value.

At block 912, "displaying the report on a display" may be performed, for example, on display 238. In some embodiments, display data for displaying the report may be generated by the processor and/or additional processors such as an image processor 236 and/or a graphics processor 240.

The systems and techniques described herein may be used with imaging data acquired using focused beams and/or diverging beams. Examples of implementing the systems and methods disclosed herein with focused and diverging beams will now be described. The examples provided herein are for exemplary purposes only and the disclosure is not limited to the examples described.

Diverging and focused beam modes based on coherent compounding may be implemented on an ultrasound imaging system, such as on the Philips S5-1 probe and EPIQ scanner. For data acquisition with diverging beams, coherent compounding of diverging beams with a frame rate of 327 frames/sec was used, whereas for focused beams, the frame rate of 196 frames/sec was used. The field of view was reduced to encompass only the interventricular septum for acquisition. A negative transmit focus (behind the probe) was chosen for diverging beams to enable harmonic imaging, which may help reduce clutter in cardiac applications. Because transmits are diverging but relatively narrow, few transmit were coherently combined, which may minimize motion artifacts during coherent compounding.

For both focused and diverging beams, a 1D cross-correlation algorithm was applied to the RF data. An example of a suitable cross-correlation algorithm is described in M. A. Lubinski, S. Y. Emelianov, and M. O'Donneil, "Speckle tracking methods for ultrasonic elasticity imaging using short-time correlation," IEEE Trans. Ultrason. Ferroelectr. Freq. Control, 1999, doi: 10.1109/58.741427. For all techniques, the myocardial mid-line in the ROI was manually traced on the first frame of the sequence, and subsequently rigidly translated and rotated to track the motion of the myocardium. ROI tracking was achieved with the Demons algorithm using a very large smoothing kernel size. An example of a suitable Demons algorithm is described in J. P. Thirion, "Image matching as a diffusion process: An analogy with Maxwell's demons," Med. Image Anal., 1998, doi: 10.1016/S1361-8415(98)80022-4. The computed radial velocities under that line were unfolded to provide a CAMM display, where the horizontal axis represents time and the vertical axis represents the distance along the myocardium. A high-pass clutter suppression filter was applied in the slow time to remove stationary clutter echoes from reverberations in the chest wall. A cut-off tissue velocity of 1cm/sec was used for the clutter filter. Tissue motion (standard RF 1D cross-correlation along the beam) was tracked twice: once with and one without preprocessing with the clutter filter, and the two resulting velocity maps were merged by keeping for each pixel the velocity with the preprocessing that locally yielded the highest cross-correlation coefficient.

Both manual tracing and a semi-automatic random sample consensus (RANSAC) algorithm to estimate the wave speed from the CAMMs was used. In manual tracing, the slope of the isovelocity wavefront was measured over a 3- to the 5-cm segment starting at the mitral annulus. Additionally, RANSAC, an iterative linear fitting method, was used to compare the robustness of the wave speed calculations.

Seventeen healthy volunteers with no history of heart failure symptoms were scanned in apical 4-chamber view. Diverging and focused beam data acquisitions were performed on all volunteers. All measurements were collected over three cardiac cycles and were repeated three times per volunteer during one scanning session. The success rate was defined as the percentage of subjects whose measured speed had a coefficient of variation <20% across all 9 measured heartbeats (the coefficient of variation of the measurements for each subject is defined by the standard deviation of that subjects' measurements divided by the average of subjects' measurements). The quality of tracking approaches was assessed qualitatively by observation of the CAMM displays.

Performance of ultrasound focused and diverging beams for the measurement of myocardial propagation speed were compared and the feasibility and reproducibility of both techniques in a group of healthy volunteers were evaluated. Over all volunteers and heartbeats, average wave speeds of 1.52 ± 0.27 m/s and 1.48 ± 0.27 m/s were measured using manual and RANSAC algorithms in the focused method; while they were 1.48 ± 0.30 m/s and 1.42 ± 0.24 m/s in diverging beams, respectively, not a significant difference. Such values are in close agreement with those velocities measured in healthy controls reported by other studies.

Both diverging and focused beam methods showed good feasibility (87.5% (15/17) and 81.25% (14/17), respectively), not a significant difference. The average across subjects of the in-subject coefficient of variation was 16.87% and 17.93%, respectively using diverging and focused beams. When focusing on the interventricular septum only, differences between diverging and focused beams were minor: even if diverging offers higher frame rate, focused beams are in general able to provide frame rates that allow adequate motion sampling of all cardiac events except valve closures. Failed cases could generally be attributed to isolated instances of the poor acoustic window as opposed to fundamental differences between the two sequences.

In some applications, the precision of the measurement can be increased by repeating the measurement over several heartbeats, as was done in the current measurements. Additionally, a decent atrial function and long heartbeat (no merging of E and A waves) are preferable for accurate myocardial propagation speed measurement.

The systems and methods disclosed herein may provide user guidance to acquire optimal images containing a cardiac tissue of interest (e.g. septum) by showing an image quality score. In some embodiments, this is performed by deep learning based model, which may learn that a viable view is defined by having entire cardiac tissue sufficiently in a B-mode image (e.g., in the middle of field-of-view) and/or sufficiently aligned with the ultrasound beams. The systems and methods disclosed herein may provide automatic ROI selection by detecting the cardiac tissue (e.g., interventricular septum). The systems and methods disclosed herein may provide automatic evaluation of frame scores within each phase and/or cardiac cycle and pass the phase and/or cardiac cycle when all frame scores within the cycle is equal to or higher than a threshold value (e.g., 0.9). The systems and methods disclosed herein may provide automatic post-processing of accepted cardiac phases and/or cycles and calculate the myocardial stretch wave speed (e.g., myocardial propagation speed) for each cycle and exclude outliers (e.g., values above or below 20% of the mean speed across all valid measurements). The systems and methods disclosed herein may provide a smart report showing all valid measurements and whisker plots of speed across all passed phases and/or cardiac cycles. In some applications, the systems and methods disclosed herein may be applied to ultrasound transducer arrays with diverging beams features (e.g., Philips Hyper2D). In some applications, the systems and methods disclosed herein may be applied to ultrasound transducer arrays with focused beam features.

In some applications, the systems and methods disclosed herein may improve accuracy and/or precision of differential HFpEF diagnosis. In some applications, the systems and methods disclosed herein may lead to more accurate and/or standardized myocardial stiffness measurements.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "C#", "Java", "Python", and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure, it is noted that the various methods and devices described herein can be implemented in hardware, software, and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instruction to perform the functions described herein.

Although the present system may have been described with particular reference to an ultrasound imaging system, it is also envisioned that the present system can be extended to other medical imaging systems where one or more images are obtained in a systematic manner. Furthermore, the principles disclosed herein are not limited to cardiac imaging. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended scope of the present system as set forth in the claims that follow. Accordingly, the specification and drawings are to be regarded in an illustrative manner and are not intended to limit the scope of the appended claims.

## Claims

1. An ultrasound imaging system (200) comprising:
an ultrasound transducer array (214) configured to acquire ultrasound images of a heart;
a display (238) configured to display the ultrasound images; and
a processor (270) configured to:
calculate, for each of the ultrasound images, an image quality score based, at least in part, on a portion of a cardiac tissue detected in each image;
record image data of a region of interest, ROI, of the cardiac tissue for ultrasound images of the heart that have a score equal to or above a predetermined threshold value during at least one phase of a cardiac cycle of the heart;
for each recorded phase, calculate a propagation speed of the cardiac tissue from the recorded image data, wherein the propagation speed is calculated from image data of the recorded phases consisting only of images having a score equal to or above the predetermined threshold value; and
provide the propagation speed on the display.

2. The ultrasound imaging system of claim 1, wherein the processor implements a neural network trained to detect the cardiac tissue in the ultrasound images, and wherein the score is based, at least in part, on an object recognition confidence metric output by the neural network.

3. The ultrasound imaging system of claim 1, wherein the processor is further configured to determine an angle of the cardiac tissue relative to an ultrasound beam transmitted by the ultrasound transducer array, and wherein the score is further based on the angle.

4. The ultrasound imaging system of claim 1, wherein the image data is processor of the region of interest is recorded as an m-mode image, wherein the processor is configured to identify an arterial kick in the m-mode image, and wherein the propagation speed is based, at least in part, on a slope of the atrial kick in the m-mode image.

5. The ultrasound imaging system of claim 1, wherein the score is further based on a signal-to-noise ratio of the ultrasound images at the cardiac wall, a frame rate of the ultrasound images, or a combination thereof.

6. The ultrasound imaging system of claim 1, wherein theat least one phase comprises at least one of an end diastolic phase or a full cardiac cycle.

7. The ultrasound imaging system of claim 1, the processor is configured to:
receive an indication of a desired number of phases or cardiac cycles for which propagation speed measurements should be calculated;
identify one or more outlier measurements form the calculated propagation speeds measurements; and
generate a report of the calculated propagation speed measurements that excludes the one or more outlier measurements.

8. The ultrasound imaging system of claim 1, further comprising an electrocardiography sensors, wherein the processor is further configured to calculate the propagation speed for ultrasound images of individual phases, wherein the phases are determined based at least in part on a signal provided by the electrocardiography sensor.

9. The ultrasound imaging system of claim 1, further comprising a user interface configured to receive a user input, wherein the user input includes a desired number of phases or cardiac cycles, wherein the processor is further configured to calculate the propagation speed for ultrasound images of individual phases or cardiac cycles until the propagation speed has been calculated for the desired number of phases or cardiac cycles.

10. A method comprising:
acquiring, by an external ultrasound imaging probe, ultrasound images of a heart;
detecting a cardiac tissue in the ultrasound images;
when the cardiac tissue is detected in the ultrasound images, analyzing the ultrasound images for a plurality of phases of one or more cardiac cycles to determine an image quality score based, at least in part, on a percentage of the cardiac tissue in the ultrasound images;
when the quality score for the ultrasound images for individual phases of the plurality of phases is equal or greater than a threshold value, calculating a propagation speed of the cardiac tissue for the individual phases;
generating a report including the propagation speeds for the individual phases; and
displaying the report on a display.

11. The method of claim 10, wherein calculating the propagation speed comprises calculating a slope of an atrial kick in an m-mode image, wherein the m-mode image is acquired along a line through the cardiac tissue.

12. The method of claim 11, further comprising segmenting the cardiac tissue from the ultrasound images and placing the line through the cardiac tissue.

13. The method of claim 11, further comprising generating a region of interest in the ultrasound images including the cardiac tissue and placing the line through a center of the region of interest.

14. A non-transitory computer-readable medium containing instructions, that when executed, cause an ultrasound imaging system to:
acquire ultrasound images of a heart;
detect a cardiac tissue in the ultrasound images;
when the cardiac tissue is detected in the ultrasound images, analyze the ultrasound images for a plurality of phases of one or more cardiac cycles to determine an image quality score based, at least in part, on a percentage of the cardiac tissue in the ultrasound images;
when the quality score for the ultrasound images for individual phases of the plurality of phases is equal or greater than a threshold value, calculate a propagation speed of the cardiac tissue for the individual phases;
generate a report including the propagation speeds for the individual phases; and
display the report on a display.

15. The non-transitory computer-readable medium of claim 14 containing instructions, that when executed, further cause the ultrasound imaging system to transmit diverging beams to acquire the ultrasound images.

## Patentansprüche

1. Ultraschallbildgebungssystem (200), umfassend:
eine Ultraschallwandleranordnung (214), die konfiguriert ist, um Ultraschallbilder eines Herzens zu erfassen;
eine Anzeige (238), die konfiguriert ist, um die Ultraschallbilder anzuzeigen; und
einen Prozessor (270), der konfiguriert ist, um:
für jedes der Ultraschallbilder, eine Bildqualitätsbewertung, die mindestens teilweise auf einem Abschnitt des in jedem Bild erkannten Herzgewebes basiert, zu berechnen;
Bilddaten eines Bereichs von Interesse des Herzgewebes für Ultraschallbilder des Herzens aufzuzeichnen, die während mindestens einer Phase eines Herzzyklus des Herzens eine Bewertung aufweisen, die gleich oder über einem vorbestimmten Schwellenwert ist;
für jede aufgezeichnete Phase eine Ausbreitungsgeschwindigkeit des Herzgewebes aus den aufgezeichneten Bilddaten zu berechnen, wobei die Ausbreitungsgeschwindigkeit aus Bilddaten der aufgezeichneten Phasen berechnet wird, die nur aus Bildern bestehen, die eine Bewertung gleich oder über dem vorgegebenen Schwellenwert aufweisen; und
die Ausbreitungsgeschwindigkeit auf der Anzeige bereitzustellen.

2. Ultraschallbildgebungssystem nach Anspruch 1, wobei der Prozessor ein neuronales Netzwerk implementiert, das trainiert ist, um das Herzgewebe in den Ultraschallbildern zu erkennen, und wobei die Bewertung mindestens teilweise auf einer vom neuronalen Netzwerk ausgegebenen Objekterkennungs-Vertrauensmetrik basiert.

3. Ultraschallbildgebungssystem nach Anspruch 1, wobei der Prozessor weiter konfiguriert ist, um einen Winkel des Herzgewebes in Bezug zu einem von der Ultraschallwandleranordnung übertragenen Ultraschallstrahl zu bestimmen, und wobei die Bewertung weiter auf dem Winkel basiert.

4. Ultraschallbildgebungssystem nach Anspruch 1, wobei die Bilddaten des Bereichs von Interesse von einem Prozessor als M-Mode-Bild aufgezeichnet werden, wobei der Prozessor konfiguriert ist, um eine Vorhofsystole in dem M-Mode-Bild zu identifizieren, und wobei die Ausbreitungsgeschwindigkeit mindestens teilweise auf einer Steigung der Vorhofsystole im M-Mode-Bild basiert.

5. Ultraschallbildgebungssystem nach Anspruch 1, wobei die Bewertung weiter auf einem Signal-Rausch-Verhältnis der Ultraschallbilder an der Herzwand, einer Einzelbildfrequenz der Ultraschallbilder oder einer Kombination davon basiert.

6. Ultraschallbildgebungssystem nach Anspruch 1, wobei die mindestens eine Phase mindestens eine enddiastolische Phase oder einen vollständigen Herzzyklus umfasst.

7. Ultraschallbildgebungssystem nach Anspruch 1, wobei der Prozessor konfiguriert ist, um:
eine Angabe einer gewünschten Anzahl von Phasen oder Herzzyklen, für die Ausbreitungsgeschwindigkeitsmessungen berechnet werden sollen, zu empfangen;
eine oder mehrere Ausreißermessungen aus den berechneten Ausbreitungsgeschwindigkeitsmessungen zu identifizieren; und
einen Bericht der berechneten Ausbreitungsgeschwindigkeitsmessungen, der die eine oder mehreren Ausreißermessungen ausschließt, zu erstellen.

8. Ultraschallbildgebungssystem nach Anspruch 1, das weiter einen Elektrokardiographiesensor umfasst, wobei der Prozessor weiter konfiguriert ist, um die Ausbreitungsgeschwindigkeit für Ultraschallbilder einzelner Phasen zu berechnen, wobei die Phasen mindestens teilweise basierend auf einem von dem Elektrokardiographiesensor bereitgestellten Signal bestimmt werden.

9. Ultraschallbildgebungssystem nach Anspruch 1, das weiter eine Benutzeroberfläche umfasst, die konfiguriert ist, um eine Benutzereingabe zu empfangen, wobei die Benutzereingabe eine gewünschte Anzahl von Phasen oder Herzzyklen beinhaltet, wobei der Prozessor weiter konfiguriert ist, um die Ausbreitungsgeschwindigkeit für Ultraschallbilder einzelner Phasen oder Herzzyklen zu berechnen, bis die Ausbreitungsgeschwindigkeit für die gewünschte Anzahl von Phasen oder Herzzyklen berechnet worden ist.

10. Verfahren, umfassend:
Erfassen durch eine externe Ultraschallbildgebungssonde, von Ultraschallbildern eines Herzens;
Erkennen eines Herzgewebes in den Ultraschallbildern;
wenn das Herzgewebe in den Ultraschallbildern erkannt wird, Analysieren der Ultraschallbilder für eine Vielzahl von Phasen eines oder mehrerer Herzzyklen, um eine Bildqualitätsbewertung zu bestimmen, die mindestens teilweise auf einem Prozentsatz des Herzgewebes in den Ultraschallbildern basiert;
wenn die Qualitätsbewertung für die Ultraschallbilder für einzelne Phasen der Vielzahl von Phasen gleich oder größer als ein Schwellenwert ist, Berechnen einer Ausbreitungsgeschwindigkeit des Herzgewebes für die einzelnen Phasen;
Erstellen eines Berichts, der die Ausbreitungsgeschwindigkeiten für die einzelnen Phasen beinhaltet; und
Anzeigen des Berichts auf einer Anzeige.

11. Verfahren nach Anspruch 10, wobei Berechnen der Ausbreitungsgeschwindigkeit Berechnen einer Steigung einer Vorhofsystole in einem M-Mode-Bild umfasst, wobei das M-Mode-Bild entlang einer Linie durch das Herzgewebe erfasst wird.

12. Verfahren nach Anspruch 11, das weiter Segmentieren des Herzgewebes anhand der Ultraschallbilder und das Platzieren der Linie durch das Herzgewebe umfasst.

13. Verfahren nach Anspruch 11, das weiter Erstellen eines Bereichs von Interesse in den Ultraschallbildern, der das Herzgewebe beinhaltet, und Platzieren der Linie durch eine Mitte des Bereichs von Interesse umfasst.

14. Nichtflüchtiges computerlesbares Medium, das Anweisungen enthält, die, wenn sie ausgeführt werden, ein Ultraschallbildgebungssystem veranlassen:
Ultraschallbilder eines Herzens zu erfassen;
ein Herzgewebe in den Ultraschallbildern zu erkennen;
wenn das Herzgewebe in den Ultraschallbildern erkannt wird, die Ultraschallbilder für eine Vielzahl von Phasen eines oder mehrerer Herzzyklen zu analysieren, um eine Bildqualitätsbewertung zu bestimmen, die mindestens teilweise auf einem Prozentsatz des Herzgewebes in den Ultraschallbildern basiert;
wenn die Qualitätsbewertung für die Ultraschallbilder für einzelne Phasen der Vielzahl von Phasen gleich oder größer als ein Schwellenwert ist, eine Ausbreitungsgeschwindigkeit des Herzgewebes für die einzelnen Phasen zu berechnen;
einen Bericht zu erstellen, der die Ausbreitungsgeschwindigkeiten für die einzelnen Phasen beinhaltet; und
den Bericht auf einer Anzeige anzuzeigen.

15. Nichtflüchtiges computerlesbares Medium nach Anspruch 14, das Anweisungen enthält, die, wenn sie ausgeführt werden, das Ultraschallbildgebungssystem weiter veranlassen, divergierende Strahlen zu übertragen, um die Ultraschallbilder zu erfassen.

## Revendications

1. Système d'imagerie à ultrasons (200) comprenant :
un réseau de transducteurs à ultrasons (214) configuré pour acquérir des images à ultrasons d'un cœur ;
un écran (238) configuré pour afficher les images à ultrasons ; et
un processeur (270) configuré pour :
calculer, pour chacune des images à ultrasons, un score de qualité d'image basé, au moins en partie, sur une partie d'un tissu cardiaque détecté dans chaque image ;
enregistrer des données d'image d'une région d'intérêt, du tissu cardiaque pour des images à ultrasons du cœur qui présentent un score égal ou supérieur à une valeur seuil prédéterminée pendant au moins une phase d'un cycle cardiaque du cœur ;
pour chaque phase enregistrée, calculer une vitesse de propagation du tissu cardiaque à partir des données d'image enregistrées, dans lequel la vitesse de propagation est calculée à partir de données d'image des phases enregistrées consistant uniquement en des images présentant un score égal ou supérieur à la valeur seuil prédéterminée ; et
fournir la vitesse de propagation sur l'écran.

2. Système d'imagerie à ultrasons selon la revendication 1, dans lequel le processeur met en œuvre un réseau neuronal formé pour détecter le tissu cardiaque dans les images à ultrasons, et dans lequel le score est basé, au moins en partie, sur une mesure de confiance de reconnaissance d'objet générée par le réseau neuronal.

3. Système d'imagerie à ultrasons selon la revendication 1, dans lequel le processeur est en outre configuré pour déterminer un angle du tissu cardiaque par rapport à un faisceau ultrasonore transmis par le réseau de transducteurs ultrasonores, et dans lequel le score est en outre basé sur l'angle.

4. Système d'imagerie à ultrasons selon la revendication 1, dans lequel les données d'image du processeur de la région d'intérêt sont enregistrées sous la forme d'une image en mode m, dans lequel le processeur est configuré pour identifier un battement artériel dans l'image en mode m, et dans lequel la vitesse de propagation est basée, au moins en partie, sur une pente du battement auriculaire dans l'image en mode m.

5. Système d'imagerie à ultrasons selon la revendication 1, dans lequel le score est en outre basé sur un rapport signal/bruit des images à ultrasons au niveau de la paroi cardiaque, une fréquence de trame des images à ultrasons ou une combinaison de ceux-ci.

6. Système d'imagerie à ultrasons selon la revendication 1, dans lequel la au moins une phase comprend au moins l'un parmi une phase diastolique finale ou un cycle cardiaque complet.

7. Système d'imagerie à ultrasons selon la revendication 1, dans lequel le processeur est configuré pour :
recevoir une indication d'un nombre souhaité de phases ou de cycles cardiaques, pour lesquels les mesures de vitesse de propagation doivent être calculées ;
identifier une ou plusieurs mesures aberrantes à partir des mesures de vitesse de propagation calculées ; et
générer un rapport des mesures de vitesse de propagation calculées qui exclut les une ou plusieurs mesures aberrantes.

8. Système d'imagerie à ultrasons selon la revendication 1, comprenant en outre des capteurs d'électrocardiographie, dans lequel le processeur est en outre configuré pour calculer la vitesse de propagation pour des images à ultrasons de phases individuelles, dans lequel les phases sont déterminées sur la base au moins en partie d'un signal fourni par le capteur d'électrocardiographie.

9. Système d'imagerie à ultrasons selon la revendication 1, comprenant en outre une interface utilisateur configurée pour recevoir une entrée utilisateur, dans lequel l'entrée utilisateur inclut un nombre souhaité de phases ou de cycles cardiaques, dans lequel le processeur est en outre configuré pour calculer la vitesse de propagation pour des images à ultrasons de phases ou de cycles cardiaques individuels jusqu'à ce que la vitesse de propagation ait été calculée pour le nombre souhaité de phases ou de cycles cardiaques.

10. Procédé comprenant :
l'acquisition, par une sonde d'imagerie à ultrasons externe, d'images à ultrasons d'un cœur ;
la détection d'un tissu cardiaque dans les images à ultrasons ;
lorsque le tissu cardiaque est détecté dans les images à ultrasons, l'analyse des images à ultrasons pour une pluralité de phases des uns ou plusieurs cycles cardiaques pour déterminer un score de qualité d'image basé, au moins en partie, sur un pourcentage du tissu cardiaque dans les images à ultrasons ;
lorsque le score de qualité des images à ultrasons pour les phases individuelles de la pluralité de phases est égal ou supérieur à une valeur seuil, le calcul d'une vitesse de propagation du tissu cardiaque pour les phases individuelles ;
la génération d'un rapport incluant les vitesses de propagation des phases individuelles ; et
l'affichage du rapport sur un écran.

11. Procédé selon la revendication 10, dans lequel le calcul de la vitesse de propagation comprend le calcul d'une pente d'un battement auriculaire dans une image en mode m, dans lequel l'image en mode m est acquise le long d'une ligne à travers le tissu cardiaque.

12. Procédé selon la revendication 11, comprenant en outre la segmentation du tissu cardiaque à partir des images à ultrasons et le placement de la ligne à travers le tissu cardiaque.

13. Procédé selon la revendication 11, comprenant en outre la génération d'une région d'intérêt dans les images à ultrasons incluant le tissu cardiaque et le placement de la ligne à travers un centre de la région d'intérêt.

14. Support non transitoire lisible par ordinateur contenant des instructions qui, lorsqu'elles sont exécutées, amènent un système d'imagerie à ultrasons à :
acquérir des images à ultrasons d'un cœur ;
détecter un tissu cardiaque dans les images à ultrasons ;
lorsque le tissu cardiaque est détecté dans les images à ultrasons, analyser les images à ultrasons pour une pluralité de phases des un ou plusieurs cycles cardiaques pour déterminer un score de qualité d'image basé, au moins en partie, sur un pourcentage du tissu cardiaque dans les images à ultrasons ;
lorsque le score de qualité des images à ultrasons pour les phases individuelles de la pluralité de phases est égal ou supérieur à une valeur seuil, calculer une vitesse de propagation du tissu cardiaque pour les phases individuelles ;
générer un rapport incluant les vitesses de propagation des phases individuelles ; et
afficher le rapport sur un écran.

15. Support non transitoire lisible par ordinateur selon la revendication 14 contenant des instructions qui, lorsqu'elles sont exécutées, amènent en outre le système d'imagerie à ultrasons à transmettre des faisceaux divergents pour acquérir les images à ultrasons.
